# EUROPEAN PATENT APPLICATION

(11) **EP 3 531 121 A1**
(43) Date of publication of application: **28.08.2019**
(21) Application number: 18158748.6
(22) Date of filing: 27.02.2018
(51) Int. Cl.: G01N 27/414, G01N 33/543

(54) **BIOSENSOR DEVICE**

(71) Applicant: Mobiosense Corp., Taipei City 100 (TW)
(72) Inventor: CHEN, Yu-Hao, Taipei City 104 (TW); CHOU, Sheng-Yeh, Taipei City 114 (TW)
(74) Representative: Ferreccio, Rinaldo

(57) **Abstract**

A biosensor device includes a sensing component (101). The sensing component includes a substrate (20), a sensing transistor (30), an isolation layer (40) and a main interface layer (50). The sensing transistor is formed on the substrate. The isolation layer covers the sensing transistor, has a common surface, and is formed with a first opening (41). The main interface layer is formed in the first opening, and has an interface surface used to receive molecules to be sensed. The main interface layer is electrically connected to the sensing transistor through a first outer via (43). The interface surface of the main interface layer is coplanar with the common surface of the isolation layer. The sensing component can further comprise a bottom-gate (312) connected to the top gate (322). Moreover, an additional control layer can be integrated to apply an auxiliary signal to attract or repel molecules to the main interface layer or to stir the sample solution. The biosensor device can further comprise a reference transistor.

## Description

### BACKGROUND OF THE INVENTION

### 1. Field of the Invention

The present invention relates to a sensor and, more particularly, to a biosensor device.

### 2. Description of Related Art

Biosensors are expected for measurements in biological features such as corpuscles, proteins, carbohydrates, antibodies or metal ions. Biosensors are advantageous for their high specificity, high sensitivity, and high selectivity, and are applicable in the fields of medicine, biological technology, food, agriculture and environment monitoring.

There are several types of biosensors, for example, electrochemical biosensor, semiconductor ion sensor, fiber-optic biosensor and piezoelectric quartz crystal biosensor.

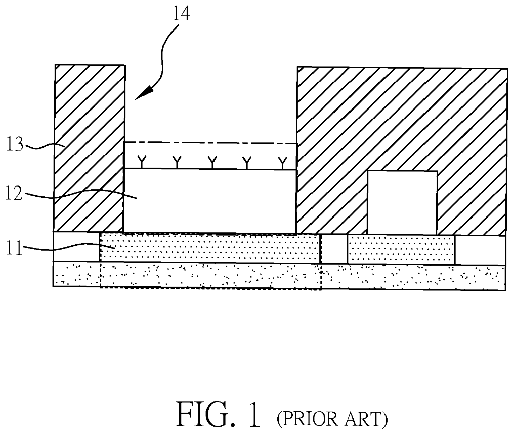

FIG. 1 shows a prior art sensor device 1. In the fabrication of the prior art sensor device 1, a silicon oxide (SiO2) layer 12 is disposed on a poly layer 11. A bulk 13 covers the SiO2 layer 12 and the poly layer 11. The bulk 13 and the SiO2 layer 12 are then etched to form a deep well 14. The poly layer 11 serves as a sensing element.

However, in one aspect, in the manufacture of the prior art sensor device 1, the bulk 13 has to be etched for a large depth which is difficult to control, and there has to be an additional etching process for the SiO2 layer 12 which cannot be etched together with the bulk 13.

Moreover, since the etching of the SiO2 layer 12 is inconsistent with the standard CMOS fabrication, it is difficult to control the remaining thickness of the SiO2 layer 12, and the yield factor of the prior art sensor device 1 varies. As a result, the prior art sensor device 1 cannot provide an accurate sensing result.

In another aspect, it takes a long time for the target matter to fall into the deep well 14 before the sensing process starts, and it is also uneasy to completely remove the target matter attached in the deep well 14 after the sensing finishes.

Therefore, it is desirable to provide an improved biosensor to mitigate or obviate the aforementioned problems.

### SUMMARY OF THE INVENTION

An object of the present invention is to provide a biosensor device that is manufactured without deep etching, or by etching a controllable depth, so as to improve manufacturing stability.

Another object of the present invention is to provide a biosensor device that is manufactured in the standard CMOS fabrication, so as to reduce the manufacturing cost.

Still another object of the present invention is to provide a biosensor device with high sensitivity by enhancing the sensing signal amplitude.

Yet another object of the present invention is to provide a biosensor device that avoids the environmental noise interference.

To achieve the objects, the biosensor device according to the present invention includes a sensing component, which further includes a substrate, a sensing transistor, an isolation layer, and a main interface layer. The sensing transistor is formed on the substrate. The isolation layer covers the sensing transistor, has a common surface and is formed with a first opening. The main interface layer is formed in the first opening and has an interface surface used to receive molecules to be sensed. The main interface layer is electrically connected to the sensing transistor through a first outer via. The interface surface of the main interface layer is coplanar with the common surface of the isolation layer.

It will be appreciated that, with the interface surface of the main interface layer coplanar with the common surface of the isolation layer, an uncontrollable deep etching is not necessary when manufacturing the biosensor device.

Moreover, the presence of the first outer via which connects the sensing transistor to the main interface layer allows the sensing transistor to be arranged at a safe distance from the molecules to be sensed. It can still avoid electrostatic discharge damage possibly caused by the molecules to be sensed even without a protection layer (such as a SiO2 layer) between the sensing transistor and the molecules to be sensed. In other words, compared with the prior art sensor device 1, the SiO2 layer 12 may be omitted, or it may be deposited with a controllable thickness rather than etched with an uncontrollable thickness.

Other objects, novel features, and their advantages of the present invention will become more apparent from the following detailed description when taken in conjunction with the accompanying drawings.

### BRIEF DESCRIPTION OF THE DRAWINGS

FIG. 1 shows a prior art sensor device;
FIG. 2 shows a block diagram of the biosensor device according to the first embodiment of the present invention;
FIG. 3 shows the sensing component of the biosensor device according to the first embodiment of the present invention;
FIG. 4 shows a perspective view of the sensing transistor according to the first embodiment of the present invention;
FIG. 5 shows the sensing component of the biosensor device according to the second embodiment of the present invention;
FIG. 6 shows the sensing component of the biosensor device according to the third embodiment of the present invention;
FIG. 7 shows the sensing component of the biosensor device according to the fourth embodiment of the present invention;
FIG. 8 shows a perspective view of the sensing transistor according to the fourth embodiment of the present invention;
FIG. 9 shows the sensing component of the biosensor device according to fifth embodiment of the present invention;
FIG. 10 shows a block diagram of the biosensor device according to the sixth embodiment of the present invention;
FIG. 11 shows the sensing component and the reference component of the biosensor device according to the sixth embodiment of the present invention;
FIG. 12 shows the sensing component of the biosensor device according to the seventh embodiment of the present invention;
FIGS. 13 to 15 show waveforms of an AC voltage, a DC voltage, and a voltage pulse, respectively.
FIG. 16 shows the sensing component of the biosensor device according to the eighth embodiment of the present invention;
FIG. 17 shows a perspective view of the structure of the first control layer, the first outer via, and the sensing transistor according to the eighth embodiment of the present invention; and
FIG. 18 shows the sensing component of the biosensor device according to the ninth embodiment of the present invention.

### DETAILED DESCRIPTION OF THE EMBODIMENTS

Different embodiments of the present invention are provided in the following description. It is to be understood that the embodiments are not meant to be limiting. Other embodiments may be utilized by arranging, substituting, combining, separating, and designing the features according to the present invention.

### First Embodiment

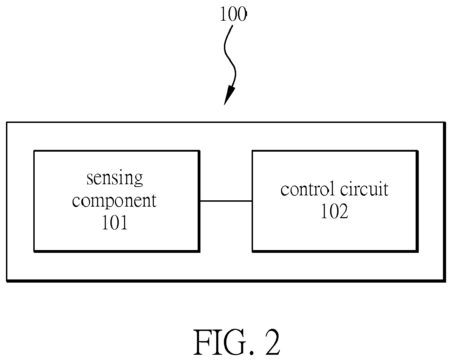

FIG. 2 shows a block diagram of the biosensor device 100 according to the first embodiment of the present invention.

### (Structure)

The biosensor device 100 includes a sensing component 101, and a control circuit 102 connected to and controls the sensing component 101.

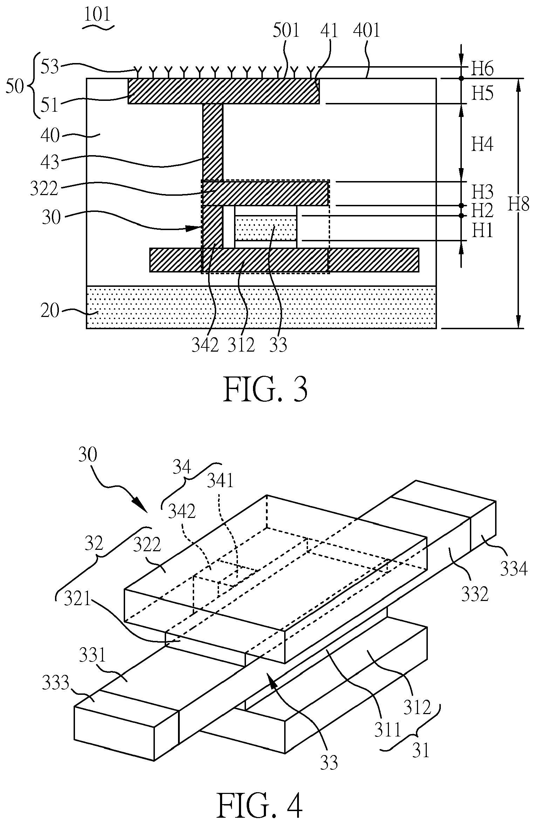

FIG. 3 shows the sensing component 101 of the biosensor device 100 according to the first embodiment of the present invention.

The sensing component 101 includes a substrate 20, a sensing transistor 30, an isolation layer 40, and a main interface layer 50.

The sensing transistor 30 is formed on the substrate 20. Optionally, the sensing transistor 30 may contact the substrate 20.

The isolation layer 40 covers the sensing transistor 30 and the substrate 20. The isolation layer 40 has a common surface 401, and is formed with a first opening 41 recessed from the common surface 401.

The main interface layer 50 is formed in the first opening 41 and has an interface surface 501. The interface surface 501 of the main interface layer 50 is coplanar with the common surface 401 of the isolation layer 40.

FIG. 4 shows a perspective view of the sensing transistor 30 according to the first embodiment of the present invention.

The sensing transistor 30 includes a bottom gate structure 31 with a bottom dielectric layer 311 and a bottom conductive layer 312, a top gate structure 32 with a top dielectric layer 321 and a top conductive layer 322, and a semiconductor layer 33.

The bottom conductive layer 312 of the bottom gate structure 31 is electrically connected to the top conductive layer 32 of the top gate structure 32 through a first inner via 34. The first inner via 34 is coated with a dielectric material 341 for electrical isolation with the semiconductor layer 33 and then filled with a conductive material 342.

The semiconductor layer 33 is electrically isolated to the bottom conductive layer 312, the top conductive layer 322 and the conductive material 342 by the bottom dielectric layer 311, the top dielectric layer 321 and the dielectric material 341, respectively.

Accordingly, the bottom gate structure 31, the top gate structure 32 and the first inner via 34 constitute a C-shaped gate structure of the sensing transistor 30.

The semiconductor layer 33 may have a thickness H1 of 3 nm to 150 nm. The bottom dielectric layer 311 or the top dielectric layer 321 may have a thickness H2 of 3 nm to 10 nm. The bottom conductive layer 312 or the top conductive layer 322 may have a thickness H3 of 3 nm to 10 nm.

It will be appreciated that, the bottom dielectric layer 311, the top dielectric layer 321 or the dielectric material 341 plays a role in protecting the semiconductor layer 33 from electrostatic discharge damage. Moreover, the manufacture of them is consistent with the standard CMOS fabrication, compared with the SiO2 layer 12 of the prior art shown in FIG. 1.

As shown in FIG. 4, the semiconductor layer 33 is shaped as a wire, and it is regarded as a semiconductor wire. However, the semiconductor layer 33 is not limited to be shaped as a wire.

In this case, the semiconductor layer 33 includes a source region 331 connected to a source electrode 333 at one end of the semiconductor wire, and a drain region 332 connected to a drain electrode 334 at the other end of the semiconductor wire. The source electrode 333 and the drain electrode 334 are further connected to the control circuit 102 shown in FIG. 2. The control circuit 102 is configured to determine the presence of the molecules to be sensed or the properties of the sensed molecules. Hereinafter, the molecules may be referred to biomolecules or their derivatives.

As shown in FIG. 4, the source region 331 and the drain region 332 may protrude beyond the region covered by the bottom gate structure 31, the top gate structure 32 and the first inner via 34. In other words, the bottom gate structure 31, the top gate structure 32 and the first inner via 34 only cover a covered portion of the semiconductor layer 33 rather than the whole semiconductor layer 33.

Referring back to FIG. 3, the main interface layer 50 is used to receive molecules to be sensed, and includes a main interface conductive layer 51 at its bottom and a receptor layer 53 at its top. In this embodiment, no protection layer such as oxide layer exists in the main interface layer 50.

The main interface conductive layer 51 is electrically connected to the top conductive layer 322 of the top gate structure 32 through a first outer via 43.

The receptor layer 53 is used to receive the molecules to be sensed by immobilizing or capturing the molecules to be sensed.

The first outer via 43 may have a longitudinal length H4 of 10 nm to 2000 nm. The main interface conductive layer 51 may be deposited with a thickness H5 of 100 nm to 1000 nm. The receptor layer 53 is deposited with a thickness H6 of 1 nm to 50 nm.

In the typical case of the thickness H6 of the receptor layer 53 being significantly small, the interface surface 501 of the main interface layer 50 may be substantially defined by the main interface conductive layer 51.

It should be emphasized that, the top surface of the main interface layer 50 is substantially coplanar with a top surface of the isolation layer 40 as shown in FIG. 3. This implies that, the isolation layer 40 only has to be etched for the thickness H5 of the main interface conductive layer 51 of the main interface layer 50 in the present invention. In other words, the first opening 41 is a shallow opening of a small depth suitable to contain the main interface layer 50. In addition, the main interface layer 50 may be formed by deposition, preferably without etching. This is advantageous in comparison with the prior art shown in FIG. 1, wherein the poly layer 11 is at the bottom of the well 14, and the bulk 13 and the SiO2 layer 12 has to be etched for a large depth which is difficult to control.

Moreover, the presence of the first outer 43 via which connects the sensing transistor 30 to the main interface layer 50 allows the sensing transistor 30 to be arranged in a safe distance from the molecules to be sensed. It can still avoid electrostatic discharge damage possibly caused by the molecules to be sensed even without a protection layer (such as a SiO2 layer) between the sensing transistor 30 and the molecules to be sensed.

Furthermore, as shown in FIG. 3, the center of the first opening 41 is not aligned with the center of the sensing transistor 30. That is, the first opening 41 is dislocated with the sensing transistor 30 from a top view of the biosensor device 100. With such dislocation, there will be additional space to contain additional components, such as a control layer which will be described below. This is advantageous in comparison with the prior art shown in FIG. 1, wherein there is no more space remained above the poly layer 11 which serves as the sensing element.

In an overall view of FIG. 3, the sensing component 101 may have a total thickness H8 less than 5 um (micrometers), wherein the total thickness H8 is defined from the bottom surface of the substrate to the common surface 401.

### (Material)

In the present invention, a semiconductor material may be Si, Ge, SiC, GaAs, GaP, InP, InAs, InSb, SiGe, GaAsP, AlInAs, AlGaAs, GaInAs, GaInP, GaInAsP, or the combinations thereof.

The substrate 20 may be made of a semiconductor material as previously described.

The semiconductor layer 33 may also be made of a semiconductor material as previously described. Moreover, without being limited to crystalline materials (such as c-Si), semiconductor materials may be polycrystalline materials (such as poly-Si) and amorphous materials (such as a-Si).

In the present invention, a dielectric material may be silicon oxide, silicon nitride, silicon oxynitride, hafnium dioxide, a dielectric with a high dielectric constant (high-k), or the combinations thereof.

The bottom dielectric layer 311, the top dielectric layer 321 and the dielectric material 341 may be made of a dielectric material as previously described.

In the present invention, a conductive material may be Cu, W, Ti, Ta, Cr, Pt, Ag, Au, TiN, TaN, NiSi, CoSi, and a doped semiconductor material.

The bottom conductive layer 312, the top conductive layer 322, the conductive material 342, the first outer via 43 and the main interface conductive layer 51 may be made of a conductive material as previously described.

The receptor layer 53 may be formed by coating enzymes, antibodies, ligands, receptors, peptides, nucleotides, cells of organs, organisms or pieces of tissue, depending on the species of the molecules to be sensed.

### (Operation)

In operation, the molecules to be sensed will induce an electric field on the main interface layer 50 when being received by the receptor layer 53, and the electric field will cause changes in electrical properties such as voltage, current, capacitance or resistance on the main interface conductive layer 51. The interface conductive layer 51 will then generate a gate signal to be sent to the top gate structure 32, particularly the top conductive layer 322, as well as to the bottom gate structure 31, particularly the bottom conductive layer 322 through the first inner via 34, particularly the conductive material 342.

The gate signal applied on the C-shaped gate structure composed of the bottom gate structure 31, the top gate structure 32, and the first inner via 34 will turn on the sensing transistor 30 to output a sensing signal. The sensing signal will then be sent to the control circuit 102 to determine the presence of the molecules to be sensed or the properties of the sensed molecules.

With the C-shaped gate structure, the gate signal can be uniformly applied to three sides of the semiconductor layer 33, and a stronger electrical field effect can be induced in the semiconductor layer 33. Thus, the sensitivity of the biosensor device 100 can be improved.

The main structure and the main function of the first embodiment are described as above.

Several preferable or optional features of the present invention are provided in the following description.

### Second Embodiment

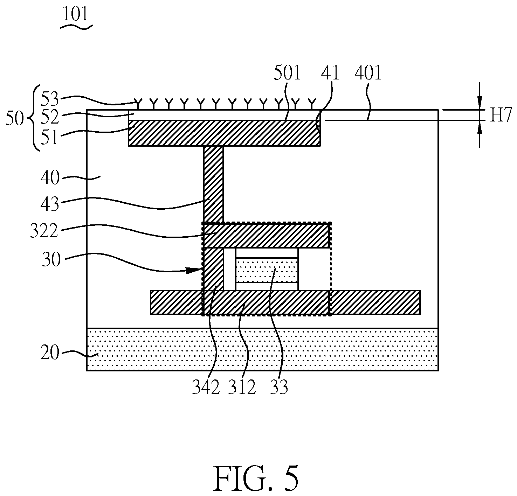

FIG. 5 shows the sensing component 101 of the biosensor device 100 according to the second embodiment of the present invention. The second embodiment is provided on the basic of the first embodiment.

In the second embodiment, the main interface layer 50 includes a main interface conductive layer 51 at its bottom, a receptor layer 53 at its top, and a main protection layer 52 disposed between the main interface conductive layer 51 and the receptor layer 53.

The main protection layer 52 may be formed by deposition, preferably without etching after the first opening 41 is formed, so that it is easier to manufacture the main protection layer 52 of the present invention than the SiO2 layer 12 of the prior art shown in FIG. 1 since the main protection layer 52 may be deposited with a controllable thickness rather than etched with an uncontrollable thickness.

The main protection layer 52 is used to protect the main interface conductive layer 51 from chemical erosion or electrostatic discharge damage, and may be made of an aforementioned conductive material or preferably an aforementioned dielectric material. The main protection layer 52 may have a thickness H7 of 3 nm to 10 nm, preferably similar to the thickness H2 of the bottom dielectric layer 311 or the top dielectric layer 321, so that all dielectric layers can be manufactured by a similar parameter consistent with the standard CMOS fabrication.

In the case of the thickness H6 of the receptor layer 53 being significantly small, the interface surface 501 of the main interface layer 50 may be substantially defined by the main protection layer 52 in this embodiment.

### Third Embodiment

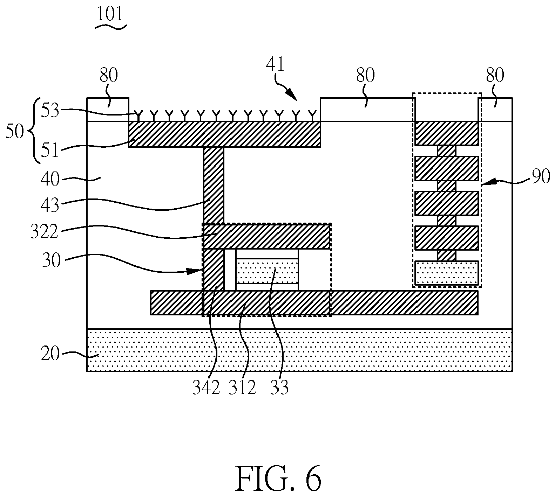

FIG. 6 shows the sensing component 101 of the biosensor device 100 according to the third embodiment of the present invention. The third embodiment is provided on the basic of the first embodiment.

In the third embodiment, the sensing component 101 includes an insulating wall layer 80 surrounding the first opening 41. The insulating wall 80 may be made of an aforementioned dielectric material, or it may be made of plastic such as Cytop, TEFLON or Parylene. The insulating wall layer 80 is used to hold the matter including the molecules to be sensed.

It will be appreciated that the insulating wall 80 may be formed without deep etching and its manufacture is consistent with the standard CMOS fabrication.

Moreover, the sensing component 101 includes a multilayer interconnection (MLI) structure 90, and a part of the isolation layer 40 is included in the multilayer interconnection structure 90. A part of the firs inner via 43 and a part of the main interface conductive layer 41 may also be included in the multilayer interconnection structure 90 since its manufacture is consistent with the standard CMOS fabrication.

### Fourth Embodiment

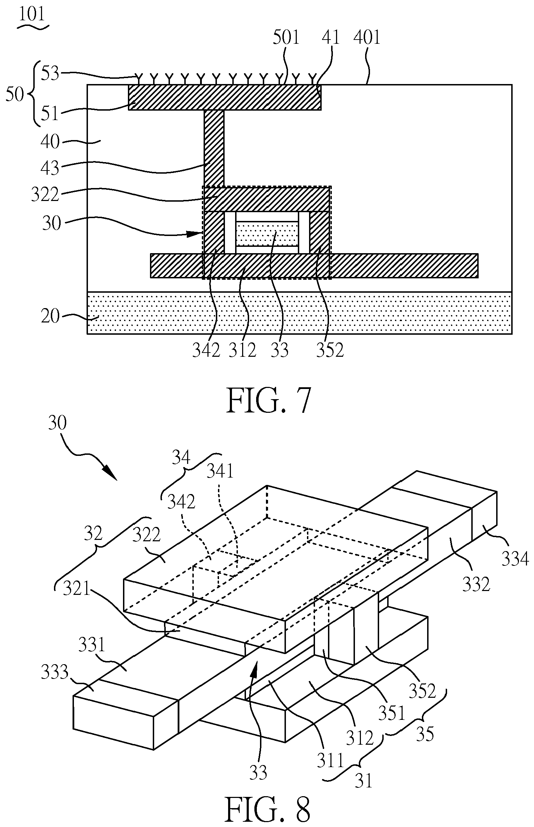

FIG. 7 shows the sensing component 101 of the biosensor device 100 according to the fourth embodiment of the present invention. The fourth embodiment is provided on the basic of the first embodiment.

In the fourth embodiment, the bottom conductive layer 312 is electrically connected to the top conductive layer 322 through a second inner via 35 in addition to the first inner via 34.

The second inner via 35 is coated with a dielectric material 351 and then filled with a conductive material 352.

Accordingly, the bottom gate structure 31, the top gate structure 32, the first inner via 34 and the second inner via 35 vertically surround the semiconductor layer 33, and constitute a surrounding gate structure of the sensing transistor 30.

FIG. 8 shows a perspective view of the sensing transistor 30 according to the fourth embodiment of the present invention. The structure of the sensing transistor 30 in FIG. 8 is similar to the structure of the sensing transistor 30 in FIG. 4, except for the presence of the second inner via 35.

In this embodiment, the bottom gate structure 31, the top gate structure 32, the first inner via 34 and the second inner via 35 only surround a surrounded portion of the semiconductor layer 33.

The fourth embodiment is advantageous in that, since the bottom conductive layer 312 and the top conductive layer 322 are connected through two paths, the first inner via 34 and the second inner via 35, the resistance of the connection between the bottom conductive layer 312 and the top conductive layer 322 is reduced, and the amplitude of the gate signal is enhanced.

Moreover, with the surrounding gate structure composed of the bottom gate structure 31, the top gate structure 32, the first inner via 34 and the second inner via 35, the gate signal can be uniformly applied to four sides of the semiconductor layer 33, and a stronger electrical field effect can be induced in the semiconductor layer 33. Thus, the sensitivity of the biosensor device 100 can be improved.

### Fifth Embodiment

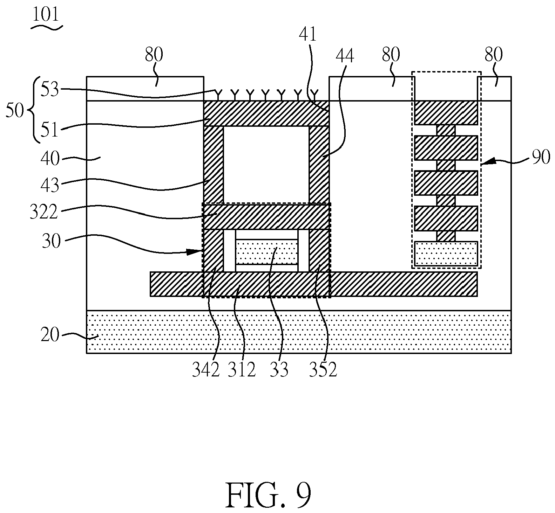

FIG. 9 shows the sensing component 101 of the biosensor device 101 according to the fifth embodiment of the present invention. The fifth embodiment is provided on the basic of the fourth embodiment.

However, in the third embodiment, the main interface conductive layer 51 is electrically connected to the top conductive layer 322 through a second outer via 44 in addition to the first outer via 43. Thus, the resistance of the connection between the main interface conductive layer 51 and the top conductive layer 322 is reduced, and the amplitude of the gate signal can be enhanced.

### Sixth Embodiment

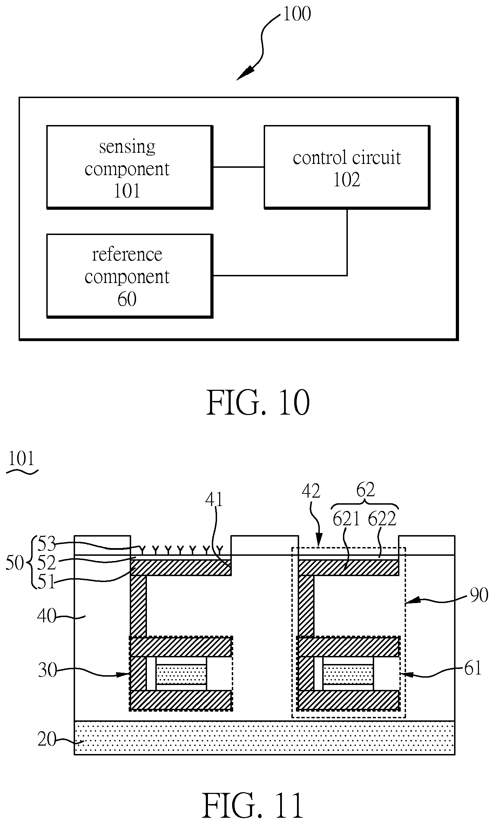

FIG. 10 shows a block diagram of the biosensor device 100 according to the sixth embodiment of the present invention. The sixth embodiment is provided on the basic of the first embodiment.

It is known that a sensing performed by a biosensor device may be interfered by environments. The same sensing performed by the same biosensor device possibly produces different results due to different environments. Herein, the environment is referred to a solution such as liquid, gas or solid which includes the molecules to be sensed. In other words, the environmental factors cause sensing inaccuracy. Thus, in the present invention, a reference component 60 is considered to be incorporated with the sensing component 101 in the biosensor device 100, in order to remove the environmental factors.

### (Structure)

FIG. 11 shows the sensing component 101 and the reference component 60 of the biosensor device 101 according to the sixth embodiment of the present invention.

In the sixth embodiment, the biosensor device 101 is further provided with a reference component 60, which includes a reference transistor 61 and a reference interface layer 62.

The reference interface layer 62 is formed on the surface of the isolation layer 40, so that it can come into touch with the environment.

The reference interface layer 62 includes a second conductive layer 621.

Optionally but preferably, the reference interface layer 62 may include a reference protection layer 622 disposed on the reference interface conductive layer 621 to protect the reference interface conductive layer 721 from chemical erosion or electrostatic discharge damage. The reference protection layer 622 may also be used to electrically isolate the interface conductive layer 721 from the molecules to be sensed.

It should be emphasized that, the reference interface layer 62 excludes a receptor layer, so that the reference interface layer 62 is incapable of immobilizing or capturing the molecules to be sensed, and thus it only senses the electric field induced by the environment rather than the electric field induced by the molecules to be sensed.

The reference interface conductive layer 621 of the reference interface layer 62 is electrically connected to a gate of the reference transistor 61 through one or more vias.

### (Operation)

In operation, the electric field induced by the environment will cause changes in electrical properties such as voltage, current, capacitance or resistance on the reference interface conductive layer 621. The reference interface conductive layer 621 then generates a reference gate signal to the gate of the reference transistor 61, turning on the reference transistor 61 to output a reference signal.

The reference signal can be compared with the sensing signal in a comparator (not shown) or a differential amplifier (not shown) which may be a part of the control circuit 102. By subtracting the reference signal from the sensing signal, the environment factors can be removed.

In order to provide a more accurate reference signal, the reference transistor 61 may be formed of similar, preferably the same structure and/or materials to the sensing transistor 30, for example, with a C-shaped gate structure or a surrounding gate structure as previously described.

The reference interface 62 may be formed of similar, preferably the same structure or materials to the main interface layer 50. For example, the reference interface layer 62 may be formed in a second opening 42 on the surface of the isolation layer 40, wherein the second opening 42 is separated from the first opening 41, and the reference interface 62 is substantially coplanar with the main interface layer 50.

Further, the reference component 60 including the reference transistor 61 and the reference interface 62 may be formed simultaneously with the sensing component including the sensing transistor 30 and the main interface layer 50 in the same manufacturing process, except that the reference interface layer 62 excludes a receptor layer.

With the similarity, the reference components 60 and the sensing components will be interfered by the environment simultaneously in a similar way, and such interference can be cancelled out.

### Seventh Embodiment

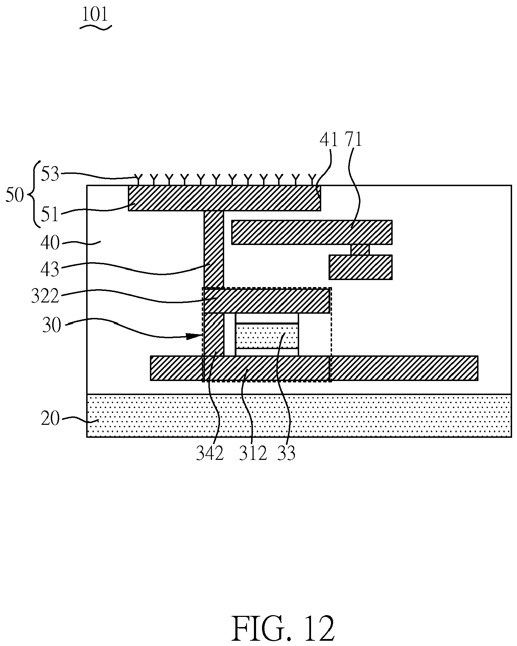

FIG. 12 shows the sensing component 101 of the biosensor device 100 according to the seventh embodiment of the present invention. The seventh embodiment is provided on the basic of the first embodiment.

### (Structure)

In the fifth embodiment, the sensing component 101 further includes a first control layer 71 under and near the first opening 41. The first control layer 71 is electrically isolated to the main interface conductive layer 51 of the main interface layer 50, so that it is also electrically isolated to the other components electrically connected to the main interface conductive layer 51, such as the top conductive layer 322.

The first control layer 71 is made of conductive materials as previously described.

### (Operation)

In operation, the first control layer 71 is applied with an auxiliary signal. The auxiliary signal may be an AC voltage, a DC voltage, or a voltage pulse. These kinds of the auxiliary signals have their respective functions as will be discussed below.

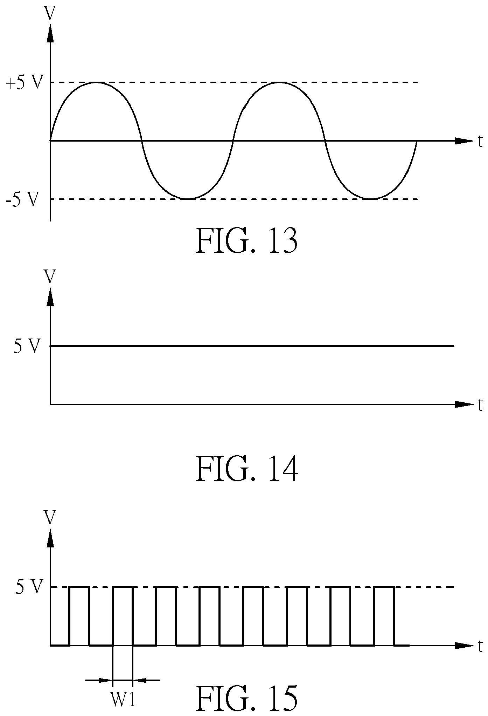

FIGS. 13 to 15 show waveforms of an AC voltage, a DC voltage, and a voltage pulse, respectively. The amplitudes of the waveforms are exemplary.

In the case of the auxiliary signal being an AC voltage, the AC voltage will induce an alternating electric field to stir a polar solution such as water. The AC voltage may have an amplitude of -5 V to +5 V and a frequency of 1kHz to 10MHz, and may be applied for 3 minutes to 15 minutes until the molecules to be sensed become uniformly distributed in the polar solution, so that the properties of the molecules will be accurately measured.

In the case of the auxiliary signal being a DC voltage, the DC voltage will induce a constant electric field to attract the molecules to be sensed, which are typically polar molecules. The DC voltage may have amplitude of 1 V to 5 V, and may be applied for 3 minutes to 15 minutes until a sufficient amount of the molecules to be sensed has fallen on the interface surface 501 of the main interface layer 50, so it will be easier to detect the presence of the molecules to be sensed in low concentration.

In the case of the auxiliary signal being a voltage pulse, the voltage pulse will induce an instant electric field to produce sufficient electric force to push the attached molecules away from the interface surface 501 of the main interface layer 50. The voltage pulse may have an amplitude of 1 V to 5 V and a pulse width W1 of 1 ns (nanosecond) to 1000 ns (nanoseconds). A consecutive series of voltage pulses may be applied for 3 minutes to 15 minutes until the attached molecules are completely removed from the interface surface 501 of the main interface layer 50.

In this embodiment, the control circuit 102 shown in FIG. 2 is also connected to the first control layer 71, and controls the first control layer 71 independently of the sensing component 101. The control circuit 102 may be configured to execute the following steps:
(a) applying an AC voltage to the first control layer 71 to stir a solution containing the molecules to be sensed;
(b) applying a DC voltage to the first control layer 71 to attract the molecules to be sensed;
(c) stopping applying any voltage to the first control layer 71; and
(d) receiving the sensing signal from the sensing transistor 30.

Optionally but preferably, the control circuit 102 may be configured to execute an addition step:
(e) applying a voltage pulse to the first control layer 71 to remove the attached molecules.

### Eighth Embodiment

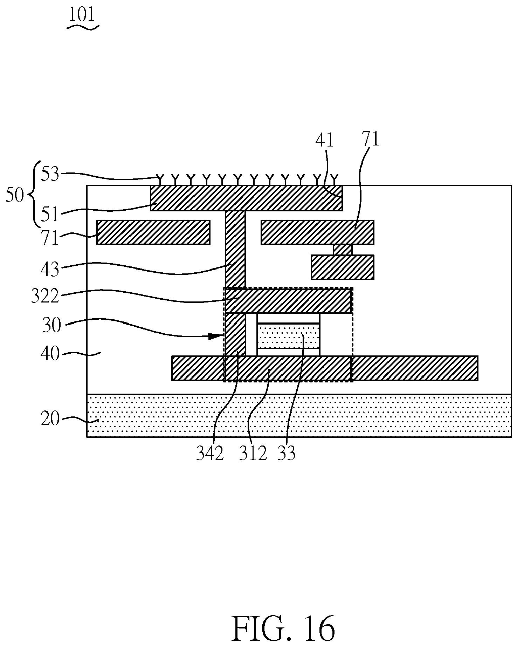

FIG. 16 shows the sensing component 101 of the biosensor device 100 according to the eighth embodiment of the present invention. The seventh embodiment is provided on the basic of the seventh embodiment.

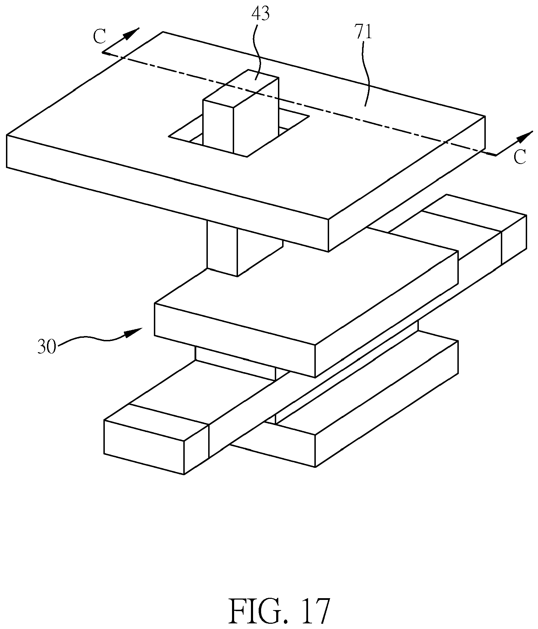

FIG. 17 shows a perspective view of the structure of the first control layer 71, the first outer via 43, and the sensing transistor 30 according to the eighth embodiment of the present invention. FIG. 16 becomes a sectional view of FIG. 17 along the line c-c.

Referring both to FIGS. 16 and 17, in this embodiment, the first control layer 71 surrounds the first outer via 43. With such arrangement, the first control layer 71 has more area overlapped with the main interface layer 50, and the auxiliary signal can efficiently affect the majority of the molecules to be sensed on the main interface layer 50.

### Ninth Embodiment

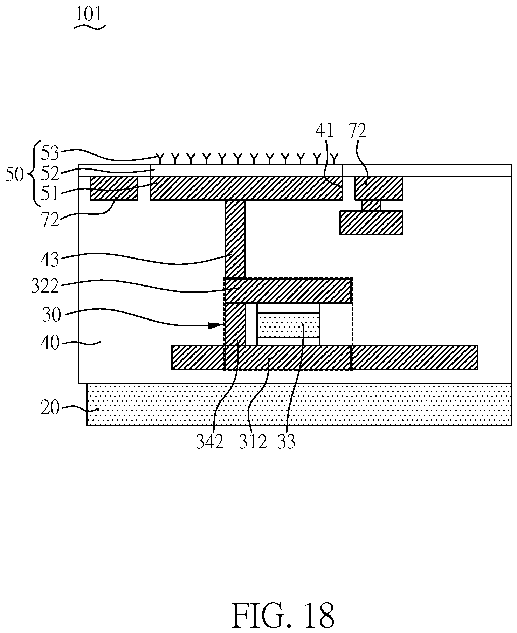

FIG. 18 shows the sensing component 101 of the biosensor device 100 according to the ninth embodiment of the present invention. The ninth embodiment is provided on the basic of the first embodiment.

### (Structure)

In the ninth embodiment, the sensing component 101 further includes a second control layer 72 surrounding and near the first opening 41. The first control layer 72 is electrically isolated to the main interface conductive layer 51 of the main interface layer 50, so that it is also electrically isolated to the other components electrically connected to the main interface conductive layer 51, such as the top conductive layer 322.

The first control layer 72 is made of a conductive material as previously described.

### (Operation)

In operation, the second control layer 72 is applied with an auxiliary signal. The auxiliary signal may be an AC voltage, a DC voltage, or a voltage pulse. These kinds of the auxiliary signals have their respective functions as will be discussed below.

FIGS. 13 to 15 show waveforms of an AC voltage, a DC voltage, and a voltage pulse, respectively.

In the case of the auxiliary signal being an AC voltage, the AC voltage will induce an alternating electric field to stir a polar solution such as water. The AC voltage may have an amplitude of -5 V to 5 V, and may be applied for 3 minutes to 15 minutes until the molecules to be sensed become uniformly distributed in the polar solution, so that the properties of the molecules to be sensed will be accurately measured.

In the case of the auxiliary signal being a DC voltage, the DC voltage will induce a constant electric field to attract the molecules to be sensed, which typically are polar molecules. The DC voltage may have amplitude of 1 V to 5 V, and may be applied for 3 minutes to 15 minutes until a sufficient amount of the molecules to be sensed has fallen on the interface surface 501 of the main interface layer 50, so it will be easier to detect the presence of the molecules to be sensed in low concentration.

In the case of the auxiliary signal being a voltage pulse, the voltage pulse will induce an instant electric field to produce sufficient electric force to push the attached molecules away from the interface surface 501 of the main interface layer 50. The voltage pulse may have an amplitude of 1 V to 5 V and a pulse width of 1 ns to 1000 ns. A consecutive series of voltage pulses may be applied for 5 minutes to 10 minutes until the attached molecules are completely removed from the interface surface 501 of the main interface layer 50.

In this embodiment, the control circuit 102 shown in FIG. 2 is also connected to the second control layer 72, and controls the second control layer 72, independently of the sensing component 101. The control circuit 102 may be configured to execute the following steps:
(a) applying an AC voltage to the second control layer 72 to stir a solution containing the molecules to be sensed;
(b) applying a DC voltage to the second control layer 72 to attract the molecules to be sensed;
(c) stopping applying any voltage to the second control layer 72; and
(d) receiving the sensing signal from the sensing transistor 30.

Optionally but preferably, the control circuit 102 may be configured to execute an addition step:
(e) applying a voltage pulse to the second control layer 72 to remove the attached molecules.

As described above, the present invention has provided a biosensor device that can improve the sensitivity by enhancing the sensing signal amplitude and removing the environmental noise interference. The biosensor device according to the present invention can be manufactured in the standard CMOS fabrication, and thus the manufacturing cost can be reduced.

Although the present invention has been explained with the aforementioned embodiment, it is to be understood the feature in one embodiment may be applicable to another embodiment, and many other modifications and variations may be made without departing from the spirit and scope of the invention as hereinafter claimed.

## Claims

1. A biosensor device (100), comprising:
a sensing component (101) including:
a substrate (20);
a sensing transistor (30) formed on the substrate (20);
an isolation layer covering (40) the sensing transistor (30), having a common surface (401) and formed with a first opening (41); and
a main interface layer (50) formed in the first opening (41) and having an interface surface (501) used to receive molecules to be sensed;
**characterized in that**:
the main interface layer (50) is electrically connected to the sensing transistor (30) through a first outer via (43); and
the interface surface (501) of the main interface layer (50) is coplanar with the common surface (401) of the isolation layer (40).

2. The biosensor device (100) as claimed in claim 1, **characterized in that**:
the sensing transistor (30) further includes a bottom gate structure (31), a top gate structure (32), and a semiconductor layer (33) formed between the bottom gate structure (31) and the top gate structure (32);
the bottom gate structure (31) is electrically connected to the top gate structure (32) through a first inner via (34) electrically isolated to the semiconductor layer (33);
the bottom gate structure (31) further includes a bottom conductive layer (312) and a bottom dielectric layer (311) disposed between the bottom conductive layer (312) and the semiconductor layer (33); and
the top gate structure (32) further includes a top conductive layer (322) and a top dielectric layer (321) disposed between the top conductive layer (322) and the semiconductor layer (33).

3. The biosensor device (100) as claimed in claims 1 or 2, **characterized in that** the main interface layer (50) includes a main interface conductive layer (51) and a receptor layer (53) formed on and contacting the main interface conductive layer (51), but excludes any oxide layer.

4. The biosensor device (100) as claimed in claims 1 or 2, **characterized in that** the main interface layer (50) includes:
a main interface conductive layer (51);
a main protection layer (52) made of a dielectric material by deposition without etching, having a thickness of 3 nm to 10 nm, formed on the main interface conductive layer (51); and
a receptor layer (53) formed on the main protection layer (52).

5. The biosensor device (100) as claimed in any of claims 1 to 4, **characterized in that** the sensing component (101) has a total thickness (H8) less than 5 um.

6. The biosensor device (100) as claimed in any of claims 1 to 5, **characterized in that** the biosensor device further comprises a first control layer (71) under the first opening (41), the first control layer (71) being electrically isolated to the main interface layer (50) and applied with an auxiliary signal.

7. The biosensor device (100) as claimed in claim 6, **characterized in that** the first control layer (71) surrounds the first outer via (43).

8. The biosensor device (100) as claimed in any of claims 1 to 7, **characterized in that** the biosensor device further comprises a second control layer (72) surrounding the first opening (41), the second control layer (72) being electrically isolated to the main interface layer (50) and applied with an auxiliary signal.

9. The biosensor device (100) as claimed in any of claims 6 to 8, **characterized in that** the auxiliary signal is an AC voltage used to stir a solution containing the molecules to be sensed.

10. The biosensor device (100) as claimed in any of claims 6 to 8, **characterized in that** the auxiliary signal is a DC voltage used to attract the molecules to be sensed.

11. The biosensor device (100) as claimed in any of claims 6 to 8, **characterized in that** the auxiliary signal is a consecutive series of voltage pulses used to remove the molecules to be sensed from the interface surface (501) of the main interface layer (50).

12. The biosensor device (100) as claimed in any of claims 6 to 11, **characterized in that** the auxiliary signal is applied for 3 minutes to 15 minutes.

13. The biosensor device (100) as claimed in any of claims 6 to 7, **characterized in that** the biosensor device further comprises a control circuit (102) connected to the sensing transistor (30) and the first control layer (71); the control circuit (102) is configured to:
apply an AC voltage to the first control layer (71) to stir a solution containing the molecules to be sensed;
apply a DC voltage to the first control layer (71) to attract the molecules to be sensed;
stop applying any voltage to the first control layer (71); and
receive the sensing signal from the sensing transistor (30).

14. The biosensor device (100) as claimed in any of claims 1 to 13, **characterized in that** the biosensor device further comprises:
a second opening (42) formed on the isolation layer (40) and separated from the first opening (41);
a reference interface layer (62) without receptors formed in the second opening (42); the reference interface layer (62) being coplanar with the main interface layer (50); and
a reference transistor (61) used to output a reference signal to be compared with the sensing signal.

15. The biosensor device (100) as claimed in claim 14, **characterized in that** the reference transistor (62) and the sensing transistor (30) are formed of a same structure.

## Amended claims

### Amended claims in accordance with Rule 137(2) EPC.

1. A biosensor device (100), comprising:
a sensing component (101) including:
a substrate (20);
a sensing transistor (30) formed on the substrate (20);
an isolation layer covering (40) the sensing transistor (30), having a common surface (401) and formed with a first opening (41);
a main interface layer (50) formed in the first opening (41) and having an interface surface (501); and
a first control layer (71) under the first opening (41), the first control layer (71) made of a conductive material, being electrically isolated to the main interface layer (50);
wherein the main interface layer (50) is electrically connected to the sensing transistor (30) through a first outer via (43); and
the interface surface (501) of the main interface layer (50) is coplanar with the common surface (401) of the isolation layer (40);
the sensing transistor (30) further includes a bottom gate structure (31), a top gate structure (32), and a semiconductor layer (33) formed between the bottom gate structure (31) and the top gate structure (32);
the bottom gate structure (31) is electrically connected to the top gate structure (32) through a first inner via (34) electrically isolated to the semiconductor layer (33);
the bottom gate structure (31) further includes a bottom conductive layer (312) and a bottom dielectric layer (311) disposed between the bottom conductive layer (312) and the semiconductor layer (33); and
the top gate structure (32) further includes a top conductive layer (322) and a top dielectric layer (321) disposed between the top conductive layer (322) and the semiconductor layer (33); and
the main interface layer (50) includes a main interface conductive layer (51), a main protection layer (52) made of a dielectric material and formed on the main interface conductive layer (51), and a receptor layer (53) formed on the main protection layer (52);
**characterized in that**:
the main protection layer (52) has a thickness of 3 nm to 10 nm; the thickness of the main protection layer (52) is similar to the thickness of the bottom dielectric layer (311) or the thickness of the top dielectric layer (321);
the first control layer (71) surrounds the first outer via (43); and
the biosensor device (100) further comprises a control circuit (102) connected to the sensing transistor (30) and the first control layer (71); the control circuit (102) is configured to apply a consecutive series of voltage pulses to the first control layer (71) to remove molecules from the interface surface (501) of the main interface layer (50), each voltage pulse having an amplitude of 1 V to 5 V and a pulse width of 1 ns to 1000 ns.

2. The biosensor device (100) as claimed in any of claims 1 to 1, **characterized in that** the sensing component (101) has a total thickness (H8) less than 5 um.

3. The biosensor device (100) as claimed in any of claims 1 and 2, **characterized in that** the control circuit (102) is configured to execute the following steps consecutively:
apply an AC voltage to the first control layer (71) to stir a solution containing the molecules to be sensed, the AC voltage having an amplitude of -5 V to +5 V and a frequency of 1kHz to 10MHz;
apply a DC voltage to the first control layer (71) to attract the molecules to be sensed, the DC voltage having an amplitude of 1 V to 5 V;
stop applying any voltage to the first control layer (71); and
receive a sensing signal from the sensing transistor (30).

4. The biosensor device (100) as claimed in any of claims 1 to 3, **characterized in that** the biosensor device further comprises:
a second opening (42) formed on the isolation layer (40) and separated from the first opening (41);
a reference interface layer (62) without receptors formed in the second opening (42); the reference interface layer (62) being coplanar with the main interface layer (50); and
a reference transistor (61) used to output a reference signal to be compared with the sensing signal.

5. The biosensor device (100) as claimed in claim 4, **characterized in that** the reference transistor (62) and the sensing transistor (30) have a same structure.
